# EUROPEAN PATENT SPECIFICATION

(11) **EP 2 510 965 B1**
(45) Date of publication and mention of the grant of the patent: **01.09.2021**
(21) Application number: 12425056.4
(22) Date of filing: 20.03.2012
(51) Int. Cl.: A61M 5/44

(54) **Device for thawing bags of frozen plasma**
Vorrichtung zum Auftauen von Beuteln mit gefrorenem Plasma
Dispositif pour la décongélation des sacs de plasma congelés

(30) Priority: 30.03.2011 IT FI20110050
(43) Date of publication of application: 17.10.2012
(73) Proprietor: KW APPARECCHI SCIENTIFICI S.R.L., 53035 Monteriggioni (SI) (IT)
(72) Inventor: Fabiani, Stefano, 53100 Siena (IT)
(74) Representative: Mannucci, Michele

(56) References cited:
- US-A- 4 486 389
- US-A- 5 243 833
- US-B1- 6 748 164

## Description

### Technical Field

The present invention relates to systems for thawing and heating medical products, and more in particular to a device for thawing and heating medical products such as bags of plasma or frozen blood or frozen stem cells, and a method for thawing and heating these products.

### State of the Art

As it is well known, to address the lack of plasma for transfusions or to use stem cells in transplants, it has been common practice at health care facilities to make stores by freezing (typically between -20°C and -40°C) single bags of plasma for subsequent use. When plasma is required, it is sufficient to thaw the necessary bags, heating them to the use temperature, typically the human body temperature (35°C - 37°C).

There are various machines for thawing and heating bags of plasma. The most used machines provide for inserting bags of plasma in a thermostatic hot water bath, heating the bag for a sufficient time to achieve the desired temperature.

However, these systems have problems linked to possible bacterial contaminations. Firstly, the bag of plasma can break and release substance inside the bath, thus contaminating it. Even more serious is the fact that a bag of plasma immersed in the bath may be contaminated with bacteria in the bath, if any. Furthermore, after thawing the operators must handle wet bags, with the risks deriving from contaminated baths. Sometimes these systems furthermore have a too slow thawing time with respect to the speed requirements in emergencies. US 6,748,164 discloses a solution to address these drawbacks; it provides for folding the bags inside heat conductive bladders, formed internally by a sealed room into which pressurized heating water is inserted. The water moves along a path inside all the bladders, that are connected in series, thus expanding and contracting them, that perform a "massaging" movement on the bags and, at the same time, transfer heat to them. The bags, presenting a mainly planar extension, are placed with this planar extension according to a horizontal arrangement on a plate oscillating around a horizontal axis; this movement facilitates thawing. After having finished its path inside the bladders, the pressurized heating water is conveyed to a drain area. The hydraulic circuit is single for all the bladders, and they thaw all the bags contemporaneously. It is not therefore possible to differentiate the heat cycles according to specific requirements.

These machines allow thawing the bag of plasma without wetting it with the heating water, but they are structurally complex and uncomfortable to be used; furthermore, they do not allow to achieve the optimal thawing and heating speed often required at the health care facilities. These machines require for instance particular seals to ensure the hydraulic connections between the pumping system and the bladder; furthermore, this latter is particularly expensive to be produced, due to the water sealed inner path.

US 5,243,833 describes another known device attempting to solve these problems. It is particularly uncomfortable, as it manages a single bag at a time; furthermore, it is not able to measure the correct temperature of the bags during thawing.

### Object and summary of the invention

The object of the present invention is to solve the problems of the known devices for thawing bags of plasma and in particular to provide a device and a method for thawing and heating bags of blood, plasma, stem cells or the like, which is simple and allows to thaw and heat one or more bags in optimal times for each bag, avoiding at the same time problems due to the contamination of the bags to be thawed, excessive heat shock of the thawed product etc.

These and other objects, that will be more apparent below, are achieved through a device according to the following claim 1 and a method according to the following claim 15.

According to a first aspect, the invention relates to a device for thawing and heating medical products such as bags of plasma or frozen blood or stem cells, comprising a main tank (provided preferably with insulated walls) for containing heating liquid, such as preferably water, provided with heating means for heating the liquid, such as a heating resistor; the device furthermore comprises more secondary small tanks (preferably with substantially rigid walls), each of which in fluid communication with the main tank, i.e. such that the liquid of the main tank can achieve the secondary small tank and/or vice versa; the device according to the invention furthermore comprises, for each secondary small tank, a pouch with floppy walls, inserted in the secondary small tank; this pouch is opened upwards for inserting a bag of medical product to be thawed, for instance a bag of plasma; the device according to the invention furthermore comprises, for each secondary small tank, an hydraulic circuit provided with a first segment of duct designed to put into fluid communication said main tank with a respective secondary small tank through pumping means (for instance an hydraulic pump pumping liquid from the main tank in the secondary small tank) and a second segment for fall of the fluid from the secondary small tank to the main tank; practically the hydraulic circuit pumps heating liquid from the main tank in the secondary small tank so that this liquid surrounds the pouch at least partially, generating a buoyancy force on the pouch making the floppy walls of the pouch adhere to the product contained inside it; through the floppy walls the liquid heat is transferred to the product, allowing thawing and subsequent heating to the desired temperature; electronic control means are advantageously provided for controlling the pumping means of each secondary small tank designed to allow filling of each secondary small tank in a way independent of that of the other secondary small tanks. This allows independent thawing and heating operations on each secondary small tank: each secondary small tank performs a heating process in a way independent of and asynchronous with respect to the other secondary small tanks, that can be set through the device control electronics. This allows a great flexibility in use, as thawing operations can be performed in different times according to the needs; the device adequately comprises one or more temperature sensors arranged in the pouch for the product to be thawed and/or heated; these sensors are preferably arranged on one or more walls of the pouch and/or on the bottom thereof. This sensor or sensors can be preferably arranged on the pouch inner face (i.e. the face into contact with the product to be thawed, that is the bag of plasma or other organic material). In other embodiments these sensors can be arranged on the pouch outer wall or between them, i.e. in the thickness of the pouch walls, always with the aim of detecting the temperature of the bag being thawed.

The device can be obviously used also to heat bags of non frozen product, i.e. simply to heat it to the desired temperature.

Obviously, the pouch does not have apertures along the walls into contact with the heating liquid, as the inside of the pouch must not be reached by the liquid. Practically, the walls (and the bottom) of the pouch designed to be surrounded by the fluid are hermetically sealed with respect to the liquid pumped in the secondary small tank, only the upper aperture being present for inserting the product to be thawed and heated. More in general, it is possible to say that the pouch is hermetically sealed at least as regards the part thereof arranged at a lower height than the maximum level of liquid achievable inside the secondary small tank.

According to a preferred embodiment, the device can comprise one or more sensors for detecting the presence of liquid that are arranged in the pouch for the product to be thawed; as in the previous case, this sensor(s) can be preferably arranged on the inner side of the pouch and more preferably a sensor for detecting the presence of liquid is arranged near the pouch bottom.

According to a preferred embodiment comprising one or more temperature sensors, described above, for detecting the temperature in the pouch, the device according to the invention can adequately comprise a storage system for storing the trend over time of the temperature detected by these temperature sensors. In this way it is possible for instance to associate each bag of plasma with its "heat history" during the thawing and/or heating step, thus allowing to assess whether that given bag has been subjected to a correct heat cycle or not and, in case, to reject it.

From a constructive viewpoint, according to a preferred embodiment the pouch for the product to be thawed and/or heated hangs in the secondary small tank. The pouch is preferably connected at its upper part with a support structure and is inserted free in the secondary small tank. This pouch is preferably connected at its upper edge, defining the aperture of the pouch, with an upper frame in correspondence of a mouth, defined on the frame, for accessing the pouch.

According to a particularly advantageous embodiment, each secondary small tank is arranged inside the main tank, with the second segment of the duct for putting into fluid communication the secondary small tank with the main tank exiting directly in the area that can be occupied by the liquid in the main tank. Each secondary small tank preferably hangs with respect to the main tank.

The main tank is preferably closed at the top by a preferably insulated covering frame, on which a mouth is obtained for accessing the pouch of each secondary small tank; each pouch is preferably fixed (hangs) at least at part of its edge (or a part near the edge) defining the aperture for inserting in the pouch the product to be thawed and/or heated, near this access mouth defined on the covering frame; also the secondary small tank is preferably connected to the covering frame of the main tank, practically hanging on it. Obviously, the secondary small tank can be for instance supported by a base structure resting on the bottom of the main tank or fixed to the walls of this latter, or fixed to the upper plane.

According to an embodiment of the device, to each pouch a cover can be associated for accessing the inside of the pouch inserted in said secondary small tank; this cover is preferably connected to the upper covering frame of the main tank or, more in general, to the structure to which the pouch is fixed, that can coincide with said covering frame.

Each secondary small tank preferably has an inlet for the heating liquid coming from the first segment of duct of fluid communication with the main tank and with which the pumping means are associated, which is arranged at a lower height with respect to the height of the fall of the liquid from the same secondary small tank. Said inlet is preferably arranged between the bottom of the respective secondary small tank and the outside of the bottom of the respective pouch contained inside it; more preferably, this inlet is obtained on the bottom (or near it) of the same secondary small tank. This configuration allows the heating liquid to rise from the bottom towards the height of fall, surrounding the pouch uniformly and thus allowing an optimal heating action. Furthermore, this configuration allows to invert the liquid flow, preferably stopping the pump, i.e. to make the liquid exit from the inlet (obtained preferably on the bottom) of the first segment of duct, thus empting the secondary small tank. Thanks to this, in case of pump stopping the liquid does not remain into contact with the pouch and therefore it does not transfer heat to the bag of plasma, thus avoiding the pouch over-heating when the device does not work or at the end of the heating cycle.

In a preferred embodiment, the fall of the liquid from the secondary small tank in the main tank is obtained through one or more passages provided in the thickness of the walls of said secondary small tank acting as overflow level for the same secondary small tank; these passages can preferably comprise lowering in the upper edge of the secondary small tank.

From an operational viewpoint, the heating liquid enters the secondary small tank pumped from the main tank by the pumping means; the secondary small tank fills, surrounding the pouch containing the bag of plasma or product to be thawed and/or heated and making the pouch adhere to the bag thanks to the liquid buoyancy force; once it has achieved the maximum filling level in the secondary small tank, the liquid is at the height for entering the second segment of duct that allows the outflow thereof due to gravity towards the main tank (practically, in a preferred embodiment this height of entering the second segment of duct acts as an overflow level for the secondary small tank, constraining the heating liquid to flow in the main tank); filling and emptying occur contemporaneously; after the initial filling phase, the secondary small tank is therefore always full of heating liquid which is continuously drained and fed until thawing and/or heating occur.

As mentioned, it is possible to invert the liquid flow, preferably stopping the pump, i.e. to make the liquid exit from the inlet (obtained preferably on the bottom) of the first segment of duct, thus empting the secondary small tank. Alternatively filling and emptying the secondary small tank (filling must be such as to surround the walls of the pouch) with the heating liquid, it is practically possible to "massage" the product to be thawed and/or heated: practically, the heating liquid fills the secondary small tank and the buoyancy force pushes against the walls of the pouch and makes them adhere to the product contained inside it with the pressure pushing on the product; the secondary small tank is then emptied from the bottom, inverting the liquid flow coming from the first segment of duct; in this way the buoyancy force on the walls of the pouch, and therefore on the product to be thawed, ends; inverting the liquid flow again, the secondary small tank fills again and the buoyancy force acts on the product again. This alternation of buoyancy force / lack in buoyancy force on the product to be thawed causes a compression/release action that "massages" the product, facilitating thawing and homogenizing the product (generally contained in a bag).

As already mentioned, the device comprises two, three or more secondary small tanks, each of which with an its own pouch and an its own hydraulic circuit, so that the respective pumping means can transfer the heating liquid from the main tank to the corresponding secondary small tank. According to another aspect, the invention relates to a method for thawing and/or heating medical products such as bags of plasma or frozen blood or stem cells, that can be obtained through the device described above, comprising one or more cycles of the following steps:
- inserting from the top the frozen product in a pouch with floppy walls contained in turn in a secondary small tank,
- making a heating liquid continuously flow in the small tank so as to surround the pouch and so that the liquid buoyancy force makes the floppy walls of the pouch adhere to the frozen product contained in it,
- making said liquid flowing out from said secondary small tank due to gravity.

According to the preferred embodiment, in steady-state conditions, the quantity of liquid entering the secondary small tank is substantially equal to the quantity of liquid exiting from the secondary small tank.

According to a preferred embodiment, the method can provide that the step of flowing of the liquid in the secondary small tank occurs from the bottom upwards. Preferably, flowing out of the heating liquid can occur from a greater height than the height of entrance of the liquid in the secondary small tank.

According to a preferred embodiment, the method provides for storing the temperature measured in said pouch during the thawing and heating cycle of the medical product contained in it.

According to the preferred embodiment, the method provides that the heating liquid enters the small tank, defined herein "secondary" tank, pushed by pumping means sucking the liquid (at a temperature equal to or greater than that the product to be thawed and/or heated must achieve) and coming from a "main" tank; the secondary small tank fills, surrounding the pouch containing the product and making the pouch adhere to the product due to the buoyancy force of the liquid; one the liquid has achieved the maximum filling level in the secondary small tank, the liquid is at a height of fall due to gravity towards the main tank; at steady-state conditions, filling and emptying occur continuously, and the secondary small tank is therefore always full of heating liquid that allows thawing and/or heating. The working cycle ends preferably when the thawing and/or heating temperature is detected on the pouch.

Adequately, according to another embodiment, the method provides for a further step, called step of "massage" of the product to be thawed, i.e. an alternation of emptying and filling the secondary small tank, obtaining an alternation of compression and release on the product to be thawed due to the buoyancy force variation in the small tank. This step can be performed before or after the above described steps, or it can completely replace them.

According to another embodiment, the method provides that the above mentioned steps are performed independently on different small tanks, sucking the liquid from a common main tank.

### Brief description of the drawings

Further characteristics and advantages of the invention shall be more apparent from the description of a preferred, although not exclusive, embodiment, illustrated by way of non limiting example in the attached tables of drawings, wherein:

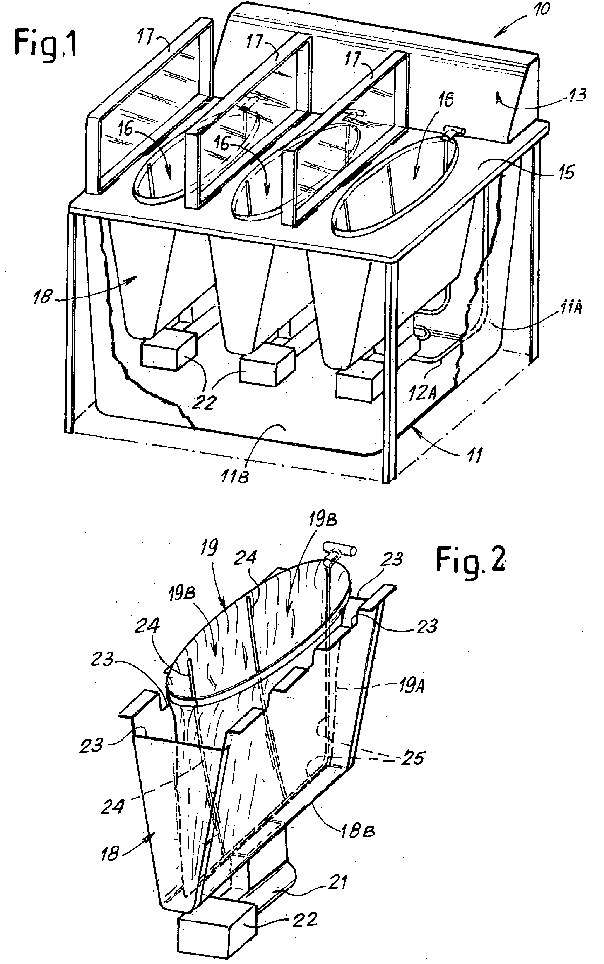

figure 1 is an axonometric schematic view of a device according to the invention;
figure 2 is a schematic axonometric view of a secondary small tank of the device of the previous figure, with the respective hydraulic circuit associated and pouch with floppy walls;
figure 3 is an axonometric view of the device of figure 1 vertically cut away according to a front plane passing through an intermediate area of the main tank;
figure 4 is a schematic side view of the device of the previous figures, cut away according to a median plane passing through a secondary small tank.

### Detailed description of an embodiment of the invention

With reference to the above cited figures, a device for thawing and heating bags of plasma according to the invention is indicated as a whole with number 10.

This device comprises a main tank 11 formed by side walls 11A and a bottom 11B that are insulated, i.e. produced in heat insulating materials so as to reduce the heat transfer from the inside towards the outside of the tank.

This main tank 11 is designed to contain the liquid acting as heating fluid and allowing to obtain thawing and heating of the bags of plasma; in this example the liquid is water. Inside the main tank 11, near its bottom 11B, heating means are arranged for heating water, for instance a resistor 12A. In the main tank 11 also water temperature first sensing means 12B are present, such as a temperature probe, designed to detect the temperature and communicate it to the electronic control means for controlling the device, generically indicated with number 13 (in correspondence of a control panel), which are in turn operatively into communication with the heating means 12A. The temperature of the water bath is preferably maintained constant (the bath is thermostatic) thanks to the combined action of the temperature sensors 12B and the heating means 12A.

To allow a uniform temperature in all the water bath, in the main tank 11 agitating means (not shown in the figures) are present, such as a bath agitating pump or agitating vanes. There are also a safety thermostat for the resistor, a faucet for discharging the bath water, and a valve for feeding water in the bath, all not shown in the figures but adequately associated with the main tank. One or more water level sensors in the main tank are present, indicated with the number 14.

With the main tank 11 a preferably insulated upper covering 15 is associated, on which three mouths 16 are obtained for accessing the areas for housing the bags of plasma to be thawed. A preferably insulated cover 17, that can be opened and closed when necessary, is associated with each access mouth 16. This cover 17 is hinged at an its own side to the upper covering 15.

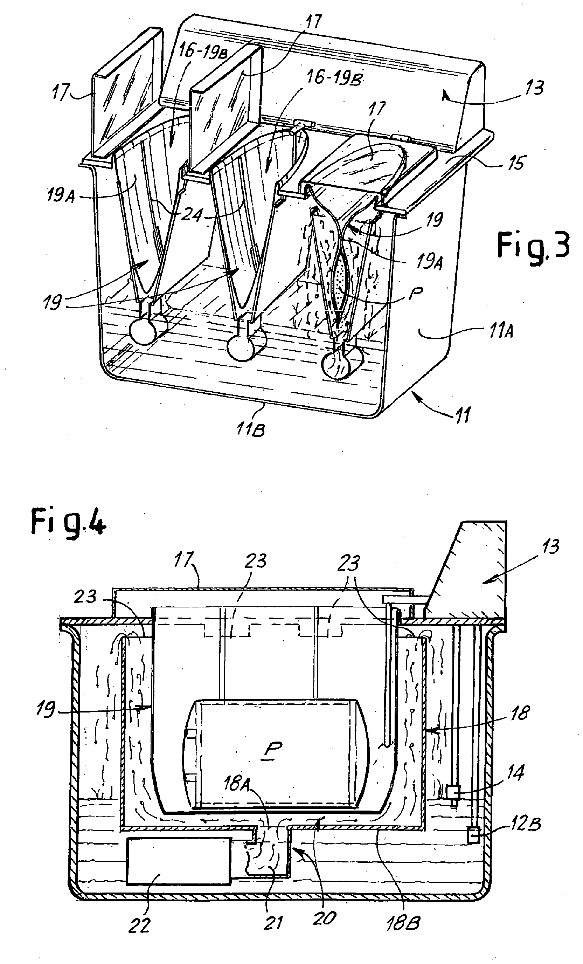

According to the invention, each area for housing one or more bags of plasma or stem cells (indicated with P in figures 3 and 4) is defined by the combination of a secondary small tank 18, opened upwards, connected at its own upper part with the covering 15 (that practically, in addition to close the main tank, acts as a support frame for the small tank) in correspondence of a respective access mouth 16, and by a pouch 19 with floppy walls 19A (or anyway semi-rigid walls, i.e. designed to transfer buoyancy force), the pouch being opened upwards through an aperture 19B for inserting a bag P of plasma; this pouch 19 is inserted and hangs in the secondary small tank 18. In this example the pouch 19 is preferably fixed in a reversible manner at its own upper edge defining the aperture 19B of the pouch to the edge of the respective access mouth 16 (practically aperture 19B of the pouch and access mouth 16 coincide). For instance, the upper edge of the aperture 19B defines a flexible or rigid flange coupling with a fixing counter-flange defined on the edge of the access mouth 16.

In this embodiment the secondary small tank 18 has advantageously a shape elongated downwards, adequately with a flared shape or a shape enlarging upwards; the walls of the secondary small tank 18 are produced with a substantially rigid material.

The overall dimensions of the part of pouch 19 inserted in the secondary small tank 18 are smaller than the inner volume defined by this latter and practically a space surrounding the pouch 19 is delimited between small tank 18 and pouch 19. The pouch (sewn and heat sealed) is produced in an impermeable material, or anyway an hermetically sealed material, with features of mechanical shear strength, abrasion resistance, traction/compression stress resistance, good deformability and chemical biological compatibility with the material of the bags for plasma and stem cells.

The device according to the invention also comprises, for each secondary small tank 18 (obviously provided with a corresponding pouch 19) an hydraulic circuit 20 provided with a first segment 21 of duct designed to put into fluid communication the main tank 11 with the respective secondary small tank 18 through pumping means, in this example comprising an hydraulic pump 22 sucking the liquid from the main tank 11 and pushing it inside the secondary small tank 18 through the first segment 21 of duct. The hydraulic circuit 20 furthermore comprises a second segment 23 of duct for fall of the water from the secondary small tank 18 to the main tank 11.

Advantageously, in this example, the inlet 18A for the water coming from the first segment 21 of duct (i.e. from the hydraulic pump 22 and, more upstream, from the main tank 11) is arranged at a lower height with respect to the height of fall of the liquid from the secondary small tank. More in particular, this inlet 18A is preferably arranged on the bottom 18B of the secondary small tank 18.

The second segment 23 of duct, from which the water falls due to gravity in the main tank 11, is practically defined by passages through the thickness of the walls thereof. These passages, also indicated with 23 for the sake of simplicity, are defined on the upper part of the secondary small tank 18, preferably near the area of fixing to the covering frame 15 of the main tank 11; these passages are defined in this example as lowering and/or slots of the upper edge of the secondary small tank 18. These passages are all defined at a same height and practically correspond to the maximum water level achievable inside the secondary small tank 18. From a practical point of view, the passages 23 correspond to an overflow level for the secondary small tank 18, as it will be better explained below.

It should be noted that the electronic control means 13 of the device provide for control means for controlling each hydraulic pump 22 designed to allow the operation of each pump independently of the others, so that each secondary small tank 18 can be filled with water independently of the others (the pumps 22 are independent of one another).

For each pouch 19 the device furthermore comprises one or more temperature second sensors 24 arranged on the face of the pouch walls facing the inside. The object of these temperature sensors 24 is continuously to monitor the temperature near the bag of plasma or stem cells being thawed/heated. The temperature second sensors 24 are operatively into communication with the electronic control means 13 of the device, with which also storing means are associated, not shown in the figures, for storing the temperature values measured over the time by these second sensors 24.

Adequately, inside each pouch 19 also one or more sensors 25 are present for detecting the presence of liquid in the pouch, preferably arranged on the bottom of the pouch and operatively into communication with the electronic control means 13 of the device.

Operation of the device, which provides a method for thawing and/or heating bags of plasma according to the invention, is as follows.

The covers 17 for accessing the pouches 19 are opened. The user inserts the bag P of frozen plasma or frozen stem cells or other frozen material inside a pouch 19 and closes the respective cover. Through the control panel associated with the device electronic control means, the user sets the thawing temperature, for instance 37°C (when the second sensors 24 will detect the thawing temperature, the device will emit an acoustical signal and the bag can be extracted). The hydraulic pump 22 is activated of the hydraulic circuit 20 of the "heating unit", comprising secondary small tank 18 and pouch 19 where the bag P (of plasma or other product) is arranged. The hot water (maintained for instance, through the resistor 12, at a higher temperature than the thawing temperature set by the user, for example comprised between 37°C and 42°C) of the main tank 11 is pumped in the secondary small tank 18 and moves from the bottom thereof upwards, surrounding the pouch 19. Thanks to the water pressure the walls of the pouch 19 adhere to the bag of plasma, as they are not rigid (see figure 3; in this figure between walls and bag P a slight space is visible, that has the only function of schematically highlighting the fact that bag and walls are two distinct elements, whilst actually the walls substantially adhere to the bag when the pouch is immersed in water). The hot water transfers heat to the bag P of plasma through the walls of the pouch 19, allowing an optimal heat transmission. The hot water continues to move upwards in the secondary small tank 18, around the pouch 19, and achieves the passages 23. Here the water "freely" falls due to gravity towards the hot water of the main tank 11 below (whose level or free-surface is, in this example and preferably, always below the inlet 18A of the first segment 21 of duct in the secondary small tank, or anyway at a lower height with respect to the bottom of the pouch 19), closing the "hydraulic circuit". Preferably, the passages 23 are obtained at such a height that the walls of the pouch completely surround the bags P of plasma of design dimensions (at least the standard dimensions of the bags of plasma and/or stem cells currently available on the market).

The hydraulic pump 22 continues to pump water inside the secondary small tank 18, so that the water inside it remains substantially always at the maximum level (as much water is pumped in the small tank, as much water falls from the small tank through the passages 23). The continuous circulation of water facilitates the heat transfer to the bag of plasma, thus heating it. The temperature second sensors 24 into contact with the surface of the bag of plasma allow to monitor and register the heating process, instant by instant.

When the surface temperature of the bag of plasma achieves the desired value the control system ends the process emptying the small tank, i.e. inverting the water flow in the first segment of duct. In this way it is possible to avid over-heating of the bag and it is possible to extract it. The emptying process occurs in few seconds.

In the final step of the process, the electronic control means can optionally empty and fill the small tank repeatedly: when the small tank is emptied, the bag is no more supported at the side by the buoyancy force and tends to modify its shape resting on the flanks of the small tank; when the secondary small tank is full the buoyancy force presses the bag again, giving it the original shape again. In this way the bag is softly massaged and the plasma is homogenized.

The bag of plasma is not into direct contact with the thermostatic bath and remains therefore completely dry and protected against contaminations.

The material forming the pouch is such as to guarantee the integrity also in case of repeated insertions of the frozen bag that can have cutting edges.

The temperature second sensor 24 inserted inside the pouch allows to track the heating process of each bag. The electronic control means 13 allow to store the temperature data. Optionally, the electronic means 13 can comprise means for interconnection to a data network and therefore the temperature data (and other data relating to the process and to the bag of frozen plasma or the bag of frozen stem cells) can be transferred in another electronic unit, or stored on adequate systems such as an SD card.

Also a barcode reader is preferably present. Each bag of plasma is adequately coded through a barcode and the device can store the information about the bag through this code; in this way it is possible subsequently to associate the temperature data detected during thawing with the coded bag of plasma. The invention relates therefore also to a system using a device according to one or more of the above illustrated combinations and bags provided with barcodes or with other identification system.

It should be noted that the device allows to actuate as many thawing processes as many are the secondary small tanks. These processes are independent from one another, i.e. they can be actuated "asynchronously" (it is possible to start the heating process of a bag when the heating process of another bag has already started: the only limit is that the temperature of the hot water bath in the main tank 11 must be always the same for both the processes).

In case of breakage of the bag of plasma, thanks to the sensors for detecting the presence of liquid it is possible to actuate an alarm sensor, allowing the operator a prompt intervention.

As mentioned, the temperature of the thermostatic bath in the main tank can be higher than the desired final temperature for the bag of plasma, as the temperature second sensors into contact with the bag indicate when heating has been performed and the electronic control system interrupts the process emptying the small tank in few seconds. In this way it is possible to reduce the duration of the process, exploiting a greater temperature differential between the heat vector and the bag with respect to the known devices.

The device according to the invention is particularly easy to be used. In fact, it is not necessary to indicate the thawing time according to the bag dimension, as it is instead necessary for the known devices, as the device automatically interrupts the process when the desired temperature is achieved. It is anyway possible for the operator to set also a thawing time.

To facilitate and improve the control, the electronic control means 13 can provide for a display allowing to show the user, in real time, the surface temperature achieved by each bag and other process control parameters.

The easy use of the device is obtained also thanks to the simple insertion of the bag from the top inside the pouch 19. The particular structure furthermore allows a high cleaning easiness: in fact, if the bag breaks, the content remains inside the pouch, without contamination of the thermostatic bath; the bag can be easily demounted, washed with running water and mounted again, furthermore maintaining the device working through the use of the other pouches.

It is understood that what illustrated purely represents possible non-limiting embodiments of the present invention, which may vary in forms and arrangements without departing from the scope of the concept on which the invention is based. Any reference numbers in the appended claims are provided for the sole purpose of facilitating the reading thereof in the light of the description hereinbefore and the accompanying drawings and do not in any way limit the scope of protection of the present invention.

## Claims

1. A device for thawing and/or heating medical products such as bags of plasma or frozen blood or stem cells or the like, comprising a main tank (11) for containing heating liquid, provided with heating means (12A) for heating the liquid contained herein, **characterized by** comprising
- a plurality of secondary small tanks (18) in fluid communication with said main tank (11),
- for each secondary small tank (18), a pouch (19) with floppy walls (19A), inserted in the respective secondary small tank (18); said pouch (19) being opened upwards for inserting a bag (P) of medical product to be thawed, temperature sensors (24) being arranged in said pouch (19),
- for each said secondary small tank (18), an hydraulic circuit (20) comprising a first segment (21) of duct designed to put into fluid communication said main tank (11) with the respective secondary small tank (18) through pumping means (22), and a second segment (23) for fall of the fluid from said secondary small tank (18) to said main tank (11); said hydraulic circuit (20) being designed to pump liquid from said main tank (11) in said respective secondary small tank (18) so that said liquid surrounds said pouch (19) at least partially, generating a buoyancy force on the pouch making the floppy walls (19A) of the pouch (19) adhere to the product (P) contained inside it,
- electronic control means (13) for controlling said pumping means (22) of each secondary small tank (18), designed to allow filling of each secondary small tank (18) in a way independent of that of the other secondary small tanks.

2. Device as claimed in claim 1, wherein each said pouch (19) hangs in the respective said secondary small tank (18).

3. Device as claimed in claim 1 or 2, wherein each secondary small tank (18) is arranged inside said main tank (11), with the respective said second segment (23) exiting directly in the area that can be occupied by the liquid in said main tank (11).

4. Device as claimed in one or more of the previous claims, wherein said first segment (21) of duct has an inlet (18A) for the heating liquid in said respective secondary small tank (18) arranged at a lower height with respect to the height of fall of the liquid from the same secondary small tank (18).

5. Device as claimed in claim 4, wherein said heating liquid inlet (18A) in a respective secondary small tank (18) is provided on the bottom (18B) of the same secondary small tank (18) or near said bottom.

6. Device as claimed in one or more of the previous claims, wherein said first segment (21) provides an hydraulic pump (22) sucking from said main tank (11) and having the discharge in fluid communication with the respective secondary small tank (18); said inlet (18A) of the first segment (21) of duct being preferably designed to discharge the liquid accumulated in the respective secondary small tank (18) when said pump (22) stops.

7. Device as claimed in one or more of the previous claims, wherein said pouch (19) is opened at the top and connected at its upper edge area to the upper part of the respective secondary small tank (18).

8. Device as claimed in one or more of the previous claims, comprising, for each secondary small tank (18), a cover (17) that can be closed for accessing the inside of the respective pouch (19) inserted in the secondary small tank (18).

9. Device as claimed in one or more of the previous claims, comprising storage means for storing the trend over time of the temperature detected by said temperature sensors (24) in said pouch.

10. Device as claimed in one or more of the previous claims, comprising sensing means (25), for detecting the presence of liquid, arranged in said pouch (19).

11. A method for thawing and/or heating medical products, such as bags of plasma or frozen blood or frozen stem cells, with a device according to claim 1, the method comprising the following steps:
- inserting from the top a plurality of frozen products in respective pouches (19) with floppy walls (19A) contained in turn in respective small tanks (18),
- making a heating liquid flow from a main tank (11) in said respective small tanks so as to surround said pouches and so that the liquid buoyancy force makes the floppy walls of the pouches adhere to the frozen products contained in them,
- making said liquid flowing out due to gravity from each small tank towards said main tank, **characterised in that** said steps are performed independently for each small tank, sucking the liquid from said common main tank.

12. Method as claimed in claim 11, wherein the step of flowing of the liquid in said small tank occurs from the bottom upwards.

13. Method as claimed in claim 11 or 12, wherein the outflow of said liquid occurs from a greater height than the height of entrance of the liquid in said small tank.

14. Method as claimed in claim 11, 12, or 13, comprising storage of the temperature in said pouch during the thawing and heating cycle of the medical product contained in it.

15. Method as claimed in one or more of claims 11 to 15, comprising a step of massage of the frozen product, presenting more sub-steps of filling of said secondary small tank, each alternating with a step of flowing out of the heating liquid from the bottom of the same secondary small tank.

## Patentansprüche

1. Vorrichtung zum Auftauen und/oder Erwärmen medizinischer Produkte, wie etwa Beutel von Plasma oder gefrorenem Blut oder Stammzellen oder dergleichen, die einen Haupttank (11) zum Enthalten von Heizflüssigkeit, versehen mit einem Erhitzungsmittel (12A) zum Erwärmen der darin enthaltenen Flüssigkeit umfasst, **gekennzeichnet durch** Umfassen von
- einer Anzahl an sekundären kleinen Tanks (18) in fluider Verbindung mit dem Haupttank (11),
- für jeden sekundären kleinen Tank (18), einer Tasche (19) mit flexiblen Wänden (19A), eingeführt in den jeweiligen sekundären kleinen Tank (18), wobei die Tasche (19) nach oben zum Einführen eines Beutels (P) eines aufzutauenden medizinischen Produkts geöffnet ist, wobei Temperatursensoren (24) in der Tasche (19) angeordnet sind,
- für jeden sekundären kleinen Tank (18), einem Hydraulickreis (20), der ein erstes Segment (21) von Rohrleitung, das ausgelegt ist, den Haupttank (11) mit dem jeweiligen sekundären kleinen Tank (18) durch ein Pumpmittel (22) in fluide Verbindung zu setzen, und ein zweites Segment (23) für Fall des Fluids aus dem sekundären kleinen Tank (18) in den Haupttank (11) umfasst; wobei der Hydraulikkreis (20) zum Pumpen von Flüssigkeit aus dem Haupttank (11) in den jeweiligen sekundären kleinen Tank (18) ausgelegt ist, sodass die Flüssigkeit die Tasche (19) mindestens teilweise umgibt und eine Auftriebskraft an der Tasche erzeugt, welche die flexiblen Wänden (19A) der Tasche (19) an dem darin enthaltenen Produkt (P) anhaften lassen,
- einem elektronischen Steuerungsmittel (13) zum Steuern des Pumpmittels (22) von jedem sekundären kleinen Tank (18), ausgelegt zum Erlauben des Füllens jedes sekundären kleinen Tanks (18) auf eine Weise, die unabhängig von jener der anderen sekundären kleinen Tanks ist.

2. Vorrichtung gemäß Anspruch 1, wobei jede Tasche (19) in dem jeweiligen sekundären kleinen Tank (18) hängt.

3. Vorrichtung gemäß Anspruch 1 oder 2, wobei jeder sekundäre kleinen Tank (18) im Inneren des Haupttanks (11) angeordnet ist, wobei das jeweilige zweite Segment (23) direkt in dem Bereich austritt, der durch die Flüssigkeit in dem Haupttank (11) eingenommen werden kann.

4. Vorrichtung gemäß einem oder mehreren der vorhergehenden Ansprüche, wobei das erste Segment (21) von Rohrleitung einen Einlass (18A) für die Heizflüssigkeit in dem jeweiligen sekundären kleinen Tank (18) aufweist, der in einer geringeren Höhe hinsichtlich der Fallhöhe der Flüssigkeit aus demselben sekundären kleinen Tank (18) angeordnet ist.

5. Vorrichtung gemäß Anspruch 4, wobei der Heizflüssigkeitseinlass (18A) in einem jeweiligen sekundären kleinen Tank (18) auf dem Boden (18B) desselben sekundären kleinen Tanks (18) oder nahe dem Boden bereitgestellt ist.

6. Vorrichtung gemäß einem oder mehreren der vorhergehenden Ansprüche, wobei das erste Segment (21) eine hydraulische Pumpe (22) bereitstellt, die aus dem Haupttank (11) saugt und den Auslass in fluider Verbindung mit dem jeweiligen sekundären kleinen Tank (18) hat; wobei der Einlass (18A) des ersten Segments (21) von Rohrleitung bevorzugt ausgelegt ist, die in dem jeweiligen sekundären kleinen Tank (18) angesammelte Flüssigkeit auszustoßen, wenn die Pumpe (22) stoppt.

7. Vorrichtung gemäß einem oder mehreren der vorhergehenden Ansprüche, wobei die Tasche (19) an der Oberseite geöffnet ist und an ihrem oberen Randbereich mit dem oberen Teil des jeweiligen sekundären kleinen Tanks (18) verbunden ist.

8. Vorrichtung gemäß einem oder mehreren der vorhergehenden Ansprüche, die für jeden sekundären kleinen Tank (18) eine Abdeckung (17) umfasst, die geschlossen werden kann, zum Zugriff auf das Innere der jeweiligen Tasche (19), die in den sekundären kleinen Tank (18) eingeführt ist.

9. Vorrichtung gemäß einem oder mehreren der vorhergehenden Ansprüche, die ein Speichermittel zum Speichern des Verlaufs der Temperatur, die durch die Temperatursensoren (24) in der Tasche detektiert wird, über die Zeit umfasst.

10. Vorrichtung gemäß einem oder mehreren der vorhergehenden Ansprüche, die ein Sensormittel (25) zum Erfassen der Anwesenheit von Flüssigkeit, angeordnet in der Tasche (19), umfasst.

11. Verfahren zum Auftauen und/oder Erwärmen medizinischer Produkte, wie etwa Beutel von Plasma oder gefrorenem Blut oder gefrorenen Stammzellen, mit einer Vorrichtung gemäß Anspruch 1, wobei das Verfahren die folgenden Schritte umfasst:
- Einführen, von der Oberseite, einer Anzahl gefrorener Produkte in jeweilige Taschen (19) mit flexiblen Wänden (19A), die wiederum in jeweiligen kleinen Tanks (18) enthalten sind,
- Bewirken, dass eine Heizflüssigkeit aus einem Haupttank (11) in die jeweiligen kleinen Tanks strömt, sodass sie die Taschen umgibt und sodass die Auftriebskraft der Flüssigkeit die flexiblen Wände der Taschen an den darin enthaltenen gefrorenen Produkten anhaften lässt,
- Ausströmenlassen der Flüssigkeit durch die Schwerkraft aus jedem kleinen Tank in Richtung des Haupttanks,
**dadurch gekennzeichnet, dass** die Schritte unabhängig für jeden kleinen Tank ausgeführt werden, wobei die Flüssigkeit aus dem gemeinsamen Haupttank gesaugt wird.

12. Verfahren gemäß Anspruch 11, wobei der Schritt des Strömens der Flüssigkeit in den kleinen Tank von dem Boden nach oben erfolgt.

13. Verfahren gemäß Anspruch 11 oder 12, wobei das Ausströmen der Flüssigkeit aus einer größeren Höhe als die Höhe des Eintritts der Flüssigkeit in den kleinen Tank erfolgt.

14. Verfahren gemäß Anspruch 11, 12 oder 13, das ein Speichern der Temperatur in der Tasche während des Auftau- und Erwärmungszyklus des darin enthaltenen medizinischen Produkts umfasst.

15. Verfahren gemäß einem oder mehreren der Ansprüche 11 bis 15, das einen Schritt der Massage des gefrorenen Produkts umfasst, der mehrere Unterschritte des Füllens des sekundären kleinen Tanks, jeweils abwechselnd mit einem Schritt des Ausströmens der Heizflüssigkeit von dem Boden desselben sekundären kleinen Tanks präsentiert.

## Revendications

1. Un dispositif pour la décongélation et/ou le réchauffage de produits médicaux tels que des sacs de plasma ou de sang congelé ou de cellules souches ou similaires, comprenant un réservoir principal (11) destiné à contenir du liquide de chauffage, pourvu de moyens de chauffage (12A) pour chauffer le liquide qu'il contient, **caractérisé en ce qu'**il comprend
- une pluralité de petits réservoirs secondaires (18) en communication fluidique avec ledit réservoir principal (11),
- pour chaque petit réservoir secondaire (18), une poche (19) avec des parois souples (19A), insérée dans le petit réservoir secondaire respectif (18) ; ladite poche (19) étant ouverte vers le haut pour l'introduction d'un sac (P) de produit médical à congeler, des capteurs de température (24) étant agencés dans ladite poche (19),
- pour chaque petit réservoir secondaire (18), un circuit hydraulique (20) comprenant un premier segment (21) de conduit destiné à mettre en communication fluidique ledit réservoir principal (11) avec le petit réservoir secondaire respectif (18) au moyen de moyens de pompage (22), et un deuxième segment (23) pour la chute du fluide dudit deuxième réservoir secondaire (18) audit réservoir principal (11) ; ledit circuit hydraulique (20) étant conçu pour pomper du liquide dudit réservoir principal (11) vers ledit petit réservoir secondaire respectif (18) de sorte que ledit liquide entoure ladite poche (19) au moins partiellement, engendrant une force de flottabilité sur la poche faisant adhérer les parois souples (19A) de la poche (19) au produit (P) contenu en son sein,
- des moyens de commande électroniques (13) pour commander lesdits moyens de pompage (22) de chaque petit réservoir secondaire (18), conçus pour permettre le remplissage de chaque petit réservoir secondaire (18) d'une manière indépendante de celle des autres petits réservoirs secondaires.

2. Dispositif selon la revendication 1, dans lequel chaque poche (19) est accrochée dans son petit réservoir secondaire respectif (18).

3. Dispositif selon la revendication 1 ou 2, dans lequel chaque petit réservoir secondaire (18) est agencé à l'intérieur dudit réservoir principal (11), avec ledit deuxième segment (23) respectif sortant directement dans la zone qui peut être occupée par le liquide dans ledit réservoir principal (11).

4. Dispositif selon l'une ou plusieurs des revendications précédentes, dans lequel ledit premier segment (21) de conduit comporte une entrée (18A) pour le liquide de chauffage dans ledit petit réservoir secondaire (18) agencé à une hauteur inférieure par rapport à la hauteur de chute du liquide depuis ce petit réservoir secondaire (18).

5. Dispositif selon la revendication 4, dans lequel ladite ouverture pour le liquide de chauffage (18A) dans un petit réservoir secondaire respectif (18) est prévue en bas (18B) de ce petit réservoir secondaire (18) ou à proximité.

6. Dispositif selon l'une ou plusieurs des revendications précédentes, dans lequel ledit premier segment (21) prévoit une pompe hydraulique (22) aspirant depuis ledit réservoir principal (11) et ayant une évacuation en communication fluidique avec le petit réservoir secondaire respectif (18) ; ladite entrée (18A) du premier segment (21) de conduit étant de préférence conçue pour évacuer le liquide accumulé dans le petit réservoir secondaire respectif (18) lorsque ladite pompe (22) s'arrête.

7. Dispositif selon l'une ou plusieurs des revendications précédentes, dans lequel ladite poche (19) est ouverte au sommet et connectée au niveau de sa zone de bord supérieure à la partie supérieure du petit réservoir secondaire respectif (18).

8. Dispositif selon l'une ou plusieurs des revendications précédentes, comprenant, pour chaque petit réservoir secondaire (18), un couvercle (17) qui peut être fermé, pour accéder à l'intérieur de la poche respective (19) insérée dans le petit réservoir secondaire (18).

9. Dispositif selon l'une ou plusieurs des revendications précédentes, comprenant des moyens d'enregistrement pour enregistrer l'évolution dans le temps de la température mesurée par lesdits capteurs de température (24) dans ladite poche.

10. Dispositif selon l'une ou plusieurs des revendications précédentes, comprenant des moyens de détection (25) pour détecter la présence de liquide, agencés dans ladite poche (19).

11. Un procédé pour décongeler et/ou réchauffer des produits médicaux, tels que des sacs de plasma ou de sang congelé ou de cellules souches congelées, avec un dispositif selon la revendication 1, ce procédé comprenant les étapes suivantes :
- l'insertion depuis le sommet d'une pluralité de produits congelés dans des poches respectives (19) ayant des parois souples (19A) contenues à leur tour dans de petits réservoirs respectifs (18),
- faire circuler un liquide de chauffage depuis un réservoir principal (11) dans lesdits petits réservoirs respectifs de manière à entourer lesdites poches et de sorte que la force de flottabilité de liquide fasse adhérer les parois souples des poches aux produits congelés contenus en leurs seins,
- faire circuler vers l'extérieur ledit liquide par gravité depuis chaque petit réservoir vers ledit réservoir principal,
**caractérisé en ce que** lesdites étapes sont effectuées indépendamment pour chaque petit réservoir, en aspirant le liquide depuis ledit réservoir principal commun.

12. Procédé selon la revendication 11, dans lequel l'étape de circulation du liquide dans ledit petit réservoir se produit de bas en haut.

13. Procédé selon la revendication 11 ou 12, dans lequel la circulation vers l'extérieur dudit liquide se produit depuis une hauteur supérieure à celle de l'entrée du liquide dans ledit petit réservoir.

14. Procédé selon la revendication 11, 12 ou 13, comprenant la circulation de la température dans ladite poche durant le cycle de décongélation et de réchauffage du produit médical contenu en son sein.

15. Procédé selon l'une ou plusieurs des revendications 11 à 15, comprenant une étape de massage du produit congelé, présentant plusieurs sous-étapes de remplissage dudit petit réservoir secondaire, chacune alternant avec une étape de circulation vers l'extérieur du liquide de chauffage depuis le bas de ce petit réservoir secondaire.
